# EUROPEAN PATENT APPLICATION

(11) **EP 4 744 589 A1**
(43) Date of publication of application: **20.05.2026**
(21) Application number: 24839866.1
(22) Date of filing: 19.04.2024
(51) Int. Cl.: A61B 6/00, A61B 6/40

(54) **PORTABLE X-RAY GENERATOR HOLDING DEVICE AND MOUNTING ASSEMBLY**

(30) Priority: 11.07.2023 KR 20230089941
(71) Applicant: Dentium Co., Ltd., Gyeonggi-do 16229 (KR)
(72) Inventor: LIM, Ji Hwan, Suwon-si, Gyeonggi-do 16229 (KR); HEO, Seong Pil, Suwon-si, Gyeonggi-do 16229 (KR); CHOO, Bum Ho, Suwon-si, Gyeonggi-do 16229 (KR)
(74) Representative: Viering, Jentschura & Partner mbB Patent- und Rechtsanwälte
(86) International application number: PCT/KR2024/005311
(87) International publication number: WO 2025/014049

(57) **Abstract**

The present disclosure provides a portable X-ray generator holding device and mounting assembly comprising: a base formed to support the lower surface of a portable X-ray generator; a side wall formed to extend from the base so as to form a surface different from the surface formed by the base, and formed to face the side surface of the portable X-ray generator; and an induction channel formed by cutting a portion from a free end of the side wall to the base, and formed to, after a handle of the portable X-ray generator enters the induction channel from the upper side of the base, induce the portable X-ray generator to a position at which the portable X-ray generator is supported on the base by gravity.

## Description

### [Technical Field]

The present disclosure relates to a portable X-ray generator holding device and a mounting assembly.

### [Background Art]

In general, an X-ray generator has been widely used in the medical field as a means of acquiring images of the inside of the human body. The X-ray generator has been manufactured in a floor-supported form (standard type). Recently, due to its ease of use, the portable X-ray generator has become widespread in dental hospitals, etc. The portable X-ray generator may be used for patients with limited mobility or during surgery.

Medical personnel may move the portable X-ray generator by holding the portable X-ray generator in their hands and irradiate patient's teeth, etc., with X-rays. The X-rays passing through teeth are detected by a separate detector, creating an image of the teeth, etc. If necessary, the portable X-ray generator may be mounted on a mounting assembly and used as a standard type.

Mounting the portable X-ray generator on the mounting assembly is not a simple process. Because the portable X-ray generators are quite heavy, there is a risk that medical personnel may drop the X-ray generator while mounting the portable X-ray generator.

Even when the portable X-ray generator is mounted on the mounting assembly, there is a risk that, when the coupling between the portable X-ray generator and the mounting assembly is not secure, the portable X-ray generator may be detached from the mounting assembly.

The above-described background art is technical information retained by the inventor to derive the embodiments of the present disclosure or acquired by the inventor while deriving the present disclosure, and thus should not be construed as art that was publicly known prior to the filing date of the present disclosure.

### [Disclosure]

### [Technical Problem]

An object of the present disclosure is to provide a portable X-ray generator holding device and a mounting assembly that enable the portable X-ray generator to be quickly coupled to the mounting assembly with minimal force.

Another object of the present disclosure is to provide a portable X-ray generator holding device and a mounting assembly that enable the portable X-ray generator to be securely coupled to the mounting assembly.

### [Technical Solution]

To achieve the above problem, according to an aspect of the present disclosure, a portable X-ray generator holding device includes: a base formed to support a lower surface of a portable X-ray generator; a side wall formed to extend from the base so as to form a surface different from a surface formed by the base, and formed to face a side surface of the portable X-ray generator; and a guide channel formed by cutting from a free end of the side wall to the base, and formed to, after a handle of the portable X-ray generator enters the guide channel from an upper side of the base, guide the portable X-ray generator to a position at which the portable X-ray generator is supported on the base by gravity.

The base may include a mounting part that surrounds a closed end portion of the guide channel and be formed concavely relative to a peripheral portion of the base.

The portable X-ray generator holding device may further include a connector electrically connected to the portable X-ray generator while the portable X-ray generator is mounted on the mounting part.

The side wall may include a first side wall connected to one end portion of the base and having the guide channel formed therein; and a second side wall connected to the other end portion of the base and disposed to face the first side wall.

The portable X-ray generator holding device may further include a retaining cover rotatably connected to the second side wall and covering an upper surface of the portable X-ray generator supported on the base to restrain the portable X-ray generator from being detached.

The portable X-ray generator holding device may further include a locking unit configured to lock the retaining cover to the first side wall.

The first side wall may include two parts divided based on the guide channel, and the locking unit may be configured to lock the two parts.

The locking unit may include: a mounting bar installed to the retaining cover and having a pair of hooks corresponding to each of the two parts; and an elastic member disposed between the mounting bar and the retaining cover to elastically support the mounting bar in a direction in which the pair of hooks are engaged with the two parts.

The locking unit may further include a frame that accommodates the mounting bar, and the elastic member may be disposed to support the mounting bar while being supported by the frame.

According to another aspect of the present disclosure, a portable X-ray generator mounting assembly includes: the portable X-ray generator holding device described above; and an arm to which the portable X-ray generator holding device is rotatably coupled.

### [Advantageous Effects]

According to a portable X-ray generator holding device and a mounting assembly according to the present disclosure configured as described above, since a guide channel for guiding the portable X-ray generator into a space defined by a base and side walls is formed by cutting from the side walls to the base, after a handle of the portable X-ray generator enters the guide channel, the portable X-ray generator is guided to a position supported on the base by gravity. As a result, the portable X-ray generator may be simply and quickly mounted on the holding device, without the cumbersome and time-consuming process of coupling the portable X-ray generator to the holding device.

Furthermore, when a retaining cover covers an upper surface of the portable X-ray generator after the portable X-ray generator is mounted within the space defined by the base and the side walls, the portable X-ray generator is more completely restrained by the holding device so that the detachment of the portable X-ray generator from the holding device may be prevented.

### [BRIEF DESCRIPTION OF THE DRAWINGS]

FIG. 1 is a perspective view illustrating a portable X-ray generator holding device 100 and a mounting assembly 200 according to an embodiment of the present disclosure.
FIG. 2 is a perspective view illustrating a state in which a portable X-ray generator X is mounted on the holding device 100 of FIG. 1.
FIG. 3 is a partial perspective view illustrating a portion of the holding device 100 of FIG. 2.
FIG. 4 is a coupled perspective view of a locking unit 190 of FIG. 2.
FIG. 5 is an exploded perspective view of the locking unit 190 of FIG. 4.

### [Best Mode]

Hereinafter, preferred embodiments of the present disclosure will be described in detail with reference to the accompanying drawings.

The present disclosure is not limited to embodiments set forth herein, but may be modified in various different forms. However, the present embodiment is provided solely to ensure that the disclosure of the present disclosure is complete and to fully inform those skilled in the art of the scope of the invention. It should be understood that the accompanying drawings are provided only in order to allow embodiments disclosed in the present specification to be easily understood, and are not intended to limit the teachings disclosed in the present specification. All the modifications, equivalents, and substitutions may be made without departing from the teachings and the scope of the present disclosure.

It should be understood that the accompanying drawings are provided only in order to allow embodiments disclosed in the present specification to be easily understood, and the teachings disclosed in the present specification are not limited by the accompanying drawings, but includes all the modifications, equivalents, and substitutions included in the teachings and the scope of the present disclosure. In the drawings, components may be exaggerated in size or thickness for ease of understanding, but this should not be construed as limiting the scope of protection of the present disclosure.

Terms used in the present specification are used only in order to describe specific implementation examples or embodiments rather than limiting the present disclosure. Singular expressions are intended to include plural expressions unless the context clearly indicates otherwise. In the specification, terms such as "~comprising," or "~consisting of" are intended to specify the presence of stated features, numbers, steps, operations, components, parts, or combinations thereof described in the specification. That is, it should be understood that terms such as "~including" or "~consisting of" in the specification do not preclude the existence or addition possibility of one or more other features or numbers, steps, operations, components, parts, or combinations thereof.

Terms including ordinal numbers such as "first," "second," etc., may be used to describe various components, but the components are not to be construed as being limited to the terms. The terms are used to distinguish one component from another component.

It is to be understood that when one element is referred to as being "connected to" or "coupled to" another element, it may be connected directly to or coupled directly to another element or be connected to or coupled to another element, having the other element intervening therebetween. On the other hand, it should be understood that when one element is referred to as being "connected directly to" or "coupled directly to" another element, it may be connected to or coupled to another element without the other element interposed therebetween.

When a component is referred to as being "above" or "below" another component, it should be understood that it is not only positioned directly above that other component, but also that other components may be present in between.

Unless indicated otherwise, it is to be understood that all the terms used in the specification including technical and scientific terms have the same meaning as those that are generally understood by those who skilled in the art. Terms generally used and defined by a dictionary should be interpreted as having the same meanings as meanings within a context of the related art and should not be interpreted as having ideal or excessively formal meanings unless being clearly defined otherwise in the present specification.

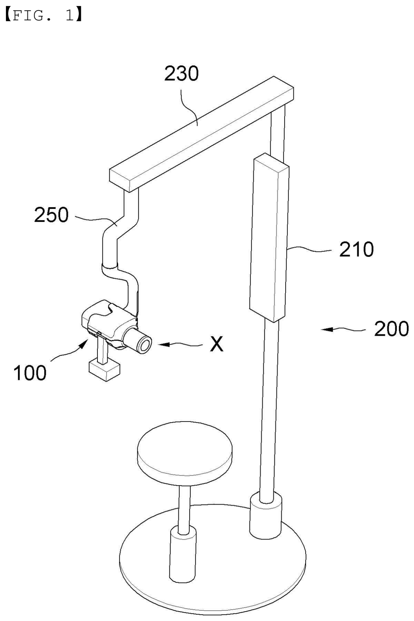

FIG. 1 is a perspective view illustrating a portable X-ray generator holding device 100 and a mounting assembly 200 according to an embodiment of the present disclosure.

Referring to this drawing, the holding device 100 holds and supports a portable X-ray generator X. The portable X-ray generator X is configured to generate X-rays and irradiate an object (such as a tooth). The X-rays passing through the object are detected by a separate detector.

The holding device 100 may also form a portion of a mounting assembly 200. The mounting assembly 200 is used while supported on the ground. The mounting assembly 200 may have a vertical bar 210 erected relative to the ground and a horizontal bar 230 extending horizontally therefrom. An arm 250 may be connected to the horizontal bar 230, and the holding device 100 may be coupled to the arm 250.

Here, the horizontal bar 230 may be rotatably coupled to the vertical bar 210. The arm 250 may also be rotatably coupled to the horizontal bar 230. The holding device 100 may also be rotatably coupled to the arm 250. By this coupling relationship, the portable X-ray generator X mounted on the mounting assembly 200 may have its position adjusted in various directions to be customized for a patient.

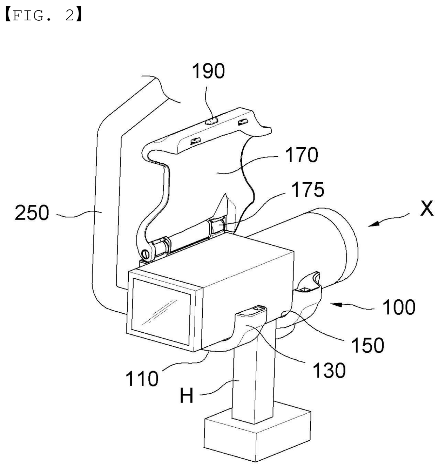

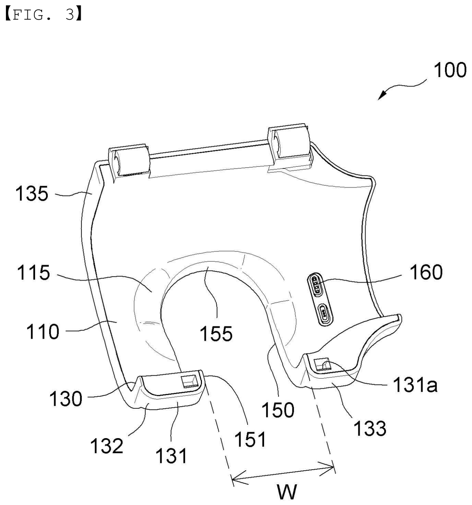

The specific configuration of the holding device 100 is described with reference to FIGS. 2 and 3. FIG. 2 is a perspective view illustrating the portable X-ray generator X mounted on the holding device 100 of FIG. 1, and FIG. 3 is a partial perspective view illustrating a portion of the holding device 100 of FIG. 2.

Referring to these drawings, the holding device 100 may include a base 110, a side wall 130, a guide channel 150, a connector 160, a retaining cover 170, and a locking unit 190.

The base 110 is formed to support a lower surface of a portable X-ray generator X. To support the portable X-ray generator X, the base 110 may have a generally flat portion.

The side wall 130 is a part that extends to form a surface different from a surface formed by the base 110. The side wall 130 may have a shape that partially or completely corresponds to an outline of the X-ray generator X, for example, a curved shape. The side wall 130 may be disposed to face a side surface of the portable X-ray generator X. Specifically, the side wall 130 may be composed of a first side wall 131 and a second side wall 135. When the first side wall 131 is connected to one end portion of the base 110, the second side wall 135 may be connected to the other end portion of the base 110. The first side wall 131 and the second side wall 135 may be disposed to face each other.

The guide channel 150 is configured to guide the portable X-ray generator X to the base 110. The guide channel 150 is formed by cutting from a free end of the side wall 130, specifically the first side wall 131, to the base 110. The guide channel 150 has a width W that allows a handle H of the portable X-ray generator X to enter.

The handle H may stop at a closed end portion 155 of the guide channel 150. The closed end portion 155 may be positioned at a point between both end portions of the base 110. At the position where the handle H stops, the portable X-ray generator X is mounted on the base 110. Specifically, the base 110 may be formed with a mounting part 115 that is concave relative to the peripheral portion. The mounting part 115 may be formed to surround the closed end portion 155. The portable X-ray generator X may be more firmly mounted on the base 110 by being positioned on the mounting part 115.

The connector 160 is configured to be electrically connected to the portable X-ray generator X. The connector 160 is mounted on the base 110. The connector 160 may be connected to the portable X-ray generator X while the portable X-ray generator X is mounted on the mounting part 115.

The retaining cover 170 is configured to cover an upper surface of the portable X-ray generator X. To this end, the retaining cover 170 may be rotatably installed on the second side wall 135. By a torsion spring (not illustrated) installed on a rotation axis 175 of the retaining cover 170, the retaining cover 170 may be elastically biased toward the first side wall 131.

The locking unit 190 is configured to lock the retaining cover 170 to the first side wall 131. The locking unit 190 may be installed on the retaining cover 170 or the first side wall 131. In this embodiment, an example in which the locking unit 190 is installed on the retaining cover 170 is illustrated.

With this configuration, medical personnel only need to insert the handle H into an open end portion 151 of the guide channel 150 while holding the handle H of the portable X-ray generator X. In this case, the portable X-ray generator X falls due to gravity from the upper side of the base 110 toward the base 110. The fall due to gravity is completed when the handle H moves from the open end portion 151 of the guide channel 150 to the closed end portion 155. In this case, the portable X-ray generator X is supported on the base 110, and a portion of the portable X-ray generator X may be more firmly mounted on the mounting part 115. Accordingly, medical personnel may simply mount the portable X-ray generator X on the holding device 100.

The portable X-ray generator X supported on the base 110 may be used in a posture in which the handle H faces downward. When the portable X-ray generator X needs to rotate so that the handle H faces sideways or upwards, it is preferable that the retaining cover 170 is closed. The retaining cover 170 covers the upper surface of the portable X-ray generator X, thereby preventing the portable X-ray generator X from being detached from the holding device 100.

The locking unit 190 reinforces a force by which the retaining cover 170 clamps the portable X-ray generator X. The retaining cover 170 is coupled to the first side wall 131 while covering the upper surface of the portable X-ray generator X, thereby preventing the retaining cover 170 from being detached from the first side wall 131.

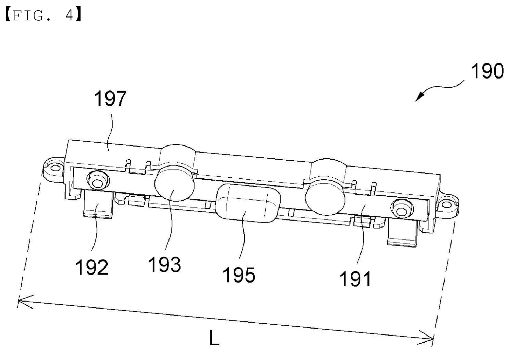

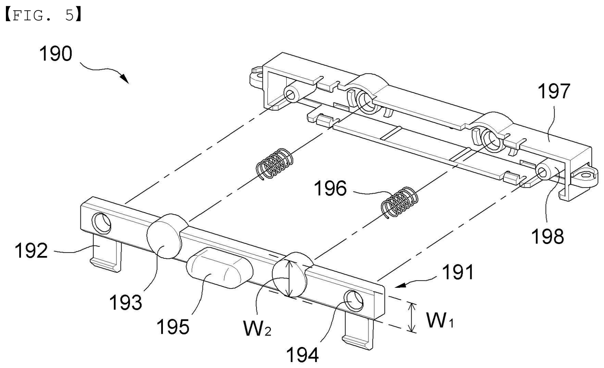

The specific configuration of the locking unit 190 is described with reference to FIGS. 4 and 5. FIG. 4 is a coupled perspective view of the locking unit 190 of FIG. 2, and FIG. 5 is an exploded perspective view of the locking unit 190 of FIG. 4.Referring further to the drawings, the first side wall 131 is divided into two parts 132 and 133 by the guide channel 150. The locking unit 190 may be formed to be locked to both parts 132 and 133. This allows for a higher fastening force between the locking unit 190 and the first side wall 131. Alternatively, when the fastening force meets a required level, the fastening between the locking unit 190 and the side wall 130 may be performed in another manner. For example, the locking unit 190 may be formed to be locked to only one of the two parts 132 and 133.

The locking unit 190 may largely include a mounting bar 191, an elastic member 196, and a frame 197.

The mounting bar 191 may be a member extending generally along a longitudinal direction. Specifically, the mounting bar 191 may have a length L greater than the width W of the guide channel 150. Hooks 192 may be provided at each of two edge regions of the mounting bar 191. The hooks 192 may be formed to be engaged with locking protrusions 131a formed on the parts 132 and 133.

An operating knob 195 may be formed at a center of the mounting bar 191. The operating knob 195 serves as a part that medical personnel press to separate the retaining cover 170 from the first side wall 131. A support part 193 may be formed between the operating knob 195 and the hook 192. The support part 193 may have a width W₂ greater than a width W₁ of other portions of the mounting bar 191.

The elastic member 196 is configured to elastically support the mounting bar 191 in a direction in which the hook 192 is engaged with the locking protrusion 131a. The elastic member 196 may be, for example, a compression coil spring. The elastic member 196 may be positioned corresponding to the support part 193.

The frame 197 is configured to accommodate the mounting bar 191. The elastic member 196 may also support the mounting bar 191 while being supported by the frame 197.

In order to guide the movement of the mounting bar 191 by the elastic member 196, a guide rod 198 may be formed to protrude from the frame 197. The guide rod 198 may be formed to extend along the direction in which the hook 192 is engaged with the locking protrusion 131a. In response to the guide rod 198, a guide hole 194 into which the guide rod 198 is inserted may be formed in the mounting bar 191.

### [Industrial Applicability]

The present disclosure has industrial applicability in the field of manufacturing a portable X-ray generator holding device and a mounting assembly.

## Claims

1. A portable X-ray generator holding device, comprising:
a base formed to support a lower surface of a portable X-ray generator;
a side wall formed to extend from the base so as to form a surface different from a surface formed by the base, and formed to face a side surface of the portable X-ray generator; and
a guide channel formed by cutting from a free end of the side wall to the base, and formed to, after a handle of the portable X-ray generator enters the guide channel from an upper side of the base, guide the portable X-ray generator to a position at which the portable X-ray generator is supported on the base by gravity.

2. The portable X-ray generator holding device of claim 1, wherein the base includes a mounting part that surrounds a closed end portion of the guide channel and is formed concavely relative to a peripheral portion of the base.

3. The portable X-ray generator holding device of claim 2, further comprising:
a connector electrically connected to the portable X-ray generator while the portable X-ray generator is mounted on the mounting part.

4. The portable X-ray generator holding device of claim 1, wherein the side wall includes:
a first side wall connected to one end portion of the base and having the guide channel formed therein; and
a second side wall connected to the other end portion of the base and disposed to face the first side wall.

5. The portable X-ray generator holding device of claim 4, further comprising:
a retaining cover rotatably connected to the second side wall and covering an upper surface of the portable X-ray generator supported on the base to restrain the portable X-ray generator from being detached.

6. The portable X-ray generator holding device of claim 5, further comprising:
a locking unit configured to lock the retaining cover to the first side wall.

7. The portable X-ray generator holding device of claim 6, wherein the first side wall includes two parts divided based on the guide channel, and
the locking unit is configured to lock the two parts.

8. The portable X-ray generator holding device of claim 7, wherein the locking unit includes:
a mounting bar installed to the retaining cover and having a pair of hooks corresponding to each of the two parts; and
an elastic member disposed between the mounting bar and the retaining cover to elastically support the mounting bar in a direction in which the pair of hooks are engaged with the two parts.

9. The portable X-ray generator holding device of claim 8, wherein the locking unit further includes a frame that accommodates the mounting bar, and
the elastic member is disposed to support the mounting bar while being supported by the frame.

10. A portable X-ray generator mounting assembly, comprising:
the portable X-ray generator holding device of claim 1; and
an arm to which the portable X-ray generator holding device is rotatably coupled.
